(19) Europäisches Patentamt / European Patent Office / Office européen des brevets

(11) EP 3 630 294 B1

(12) **EUROPEAN PATENT SPECIFICATION**

(45) Date of publication and mention of the grant of the patent: **15.11.2023 Bulletin 2023/46**

(21) Application number: **17911526.6**

(22) Date of filing: **31.05.2017**

(51) International Patent Classification (IPC):
***A61Q 5/00*** *(2006.01)* ***A61K 8/891*** *(2006.01)*
***A61K 8/898*** *(2006.01)* ***A61K 8/06*** *(2006.01)*
***A61K 8/41*** *(2006.01)* ***A61K 8/86*** *(2006.01)*
***A61Q 5/12*** *(2006.01)* ***A61K 8/34*** *(2006.01)*
***A61K 8/39*** *(2006.01)*

(52) Cooperative Patent Classification (CPC):
**A61Q 5/12; A61K 8/062; A61K 8/342; A61K 8/39; A61K 8/416; A61K 8/86; A61K 8/891; A61K 8/898**

(86) International application number:
**PCT/CN2017/086576**

(87) International publication number:
**WO 2018/218492 (06.12.2018 Gazette 2018/49)**

(54) **COMPOSITION FOR CONDITIONING HAIR**

HAARPFLEGEZUSAMMENSETZUNG

COMPOSITION DE CONDITIONNEMENT DE CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**08.04.2020 Bulletin 2020/15**

(73) Proprietors:
- **L'OREAL**
  **75008 Paris (FR)**
  Designated Contracting States:
  **AT BE BG CH CY CZ DE DK EE ES FI FR GB GR HR HU IE IS IT LI LT LU LV MC MK MT NL NO PL PT RO RS SE SI SK SM TR**
- **Liu, Hui**
  **Shanghai 201206 (CN)**
  Designated Contracting States:
  **AL**
- **Chia, Wi-Soon**
  **Shanghai 201206 (CN)**
  Designated Contracting States:
  **AL**
- **De Boni, Maxime**
  **Shanghai 201206 (CN)**
  Designated Contracting States:
  **AL**

(72) Inventors:
- **LIU, Hui**
  **Shanghai 201206 (CN)**
- **CHIA, Wi-Soon**
  **Shanghai 201206 (CN)**
- **DE BONI, Maxime**
  **Shanghai 201206 (CN)**

(74) Representative: **Casalonga**
**Casalonga & Partners**
**Bayerstraße 71/73**
**80335 München (DE)**

(56) References cited:
**WO-A1-01/01937**
**WO-A1-2015/082358**
**WO-A2-2012/152722**
**GB-A- 2 540 236**
**US-A1- 2002 182 161**
**US-A1- 2015 313 828**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 630 294 B1

## Description

The present invention relates to hair compositions, such as compositions for caring for hair, more particularly for conditioning hair. The invention also relates to a cosmetic process for conditioning hair, using this composition.

In order to improve the cosmetic properties of the compositions for application to hair, in particular sensitized hair, i.e., hair which is damaged or weakened, in particular through the chemical action of environmental agents and/or of hair treatments such as permanent-waving, dyeing or bleaching, it is known to introduce into these compositions conditioning agents. The main purpose of these conditioning agents is to rectify or limit the undesirable effects induced by the various treatments or types of attach to which the hair fibers are more or less repeatedly subjected to and, of course, they can also improve the cosmetic behavior of natural hair.

The conditioning agents most commonly used to date are cationic polymers, silicones and/or silicone derivatives which impart to washed, dry or wet hair a disentangling, softness and a smoothness which are markedly enhanced in comparison to what can be obtained with corresponding compositions which do not contain them.

It is known, for example from US6417145, to use a mixture of silicone and cationic polymer in a shampoo, to wash and condition the hair. However, these compositions still have numerous disadvantages, such as presenting a low foam power and leading to an insufficient deposit of silicones on hair impacting therefore strongly on their cosmetic properties.

It is also known, for example from US6610280, a hair treatment composition containing a silicone component comprising droplets of silicone blend, said silicone blend comprising from 50 to 95% by weight of a first silicone having a viscosity of at least 100,000 $mm^2/sec$ and from 5 to 50% by weight a second silicone which is functionalized, for example amino-functionalized silicones.

But these compositions are not completely satisfactory. The silicone emulsions currently used in personal care compositions usually comprise high viscosity silicones that give a good smoothness and feel properties but have the disadvantage of giving heavy feel and build-up on the hair, and incur problem during removal from the hair while rinsing.

Also, the emulsions that are used in the prior art are mainly big blob emulsions and hence the silicone deposition is not very uniform, and the desired performance is not achieved.

Thus, there is a real need to provide cosmetic compositions, such as compositions for conditioning hair, that do not have the combination of drawbacks described above, i.e. which can enhance cosmetic properties of said fibers, namely by affording them softness, smoothness and disentangling. The composition should trigger satisfactory silicone deposit on the keratin fibers.

The Applicant has now discovered that the use of a specific emulsion comprising silicones, especially in a conditioner base, makes it possible to achieve the objectives outlined above.

Thus, one object of the invention is especially a hair composition comprising:

(i) at least one cationic surfactant of the formula (I),

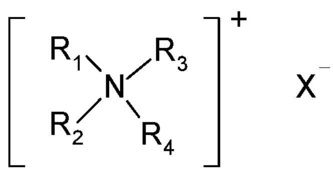

(I)

wherein:

$R_1$ to $R_4$, identical or different, represent a linear or branched aliphatic group comprising from 1 to 30 carbon atoms, an aromatic group such as aryl or alkylaryl,
at least one of the groups $R_1$ to $R_4$ comprising from 8 to 30 carbon atoms, and
$X^-$ is an anionic counterion chosen from halides;

(ii) an oil-in-water emulsion having $D_{50}$ particle size of less than 350 nm and comprising :

- a silicone mixture comprising (i) a trialkylsilyl terminated dialkylpolysiloxane having a viscosity of from 40,000 to less than 100,000 mPa.s at 25°C and (ii) an amino-silicone having a viscosity of from 1,000 to 15,000 mPa.s at 25°C and an amine value of from 2 to 10 mg of KOH per gram of amino-silicone;
- a mixture of emulsifiers comprising one or more nonionic emulsifiers, wherein the mixture of emulsifiers has a HLB value from 10 to 16; and

- water; and

(iii) at least one fatty alcohol.

Another object of the present invention is a process for the cosmetic treatment of hair, preferably for conditioning hair, comprising the steps of applying to the hair, preferably in a wet state, the hair composition described above, and optionally rinsing them with water after an optional period of exposure.

The composition according to the present invention is able to deposit a high amount of silicones on the hair, even when the composition contains a small amount of silicones.

In addition, the composition according to the invention leads to improved cosmetic properties, and especially affords good conditioning of the hair, including when the hair is sensitized. Indeed, the composition of the invention provides, for instance, smoothness, softness and disentangling to the hair.

In that which follows and unless otherwise indicated, the limits of a range of values are included within this range, in particular in the expressions "of between" and "ranging from ... to ... ". Moreover, the expression "at least one" or "at least a" used in the present description is equal to the expression "one or more".

**A - Surfactants**

The composition of the present invention comprises at least one cationic surfactant, chosen from the compound of formula (I),

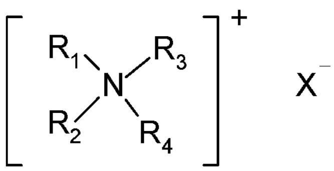

(I)

in which,

$R_1$ to $R_4$, which may be identical or different, represent a linear or branched aliphatic group comprising from 1 to 30 carbon atoms or an aromatic group such as aryl or alkylaryl, and at least one of the groups $R_1$ to $R_4$ comprising from 8 to 30 carbon atoms.

The aliphatic groups are chosen, for example, from $C_1$-$C_{30}$ alkyl, $C_1$-$C_{30}$ alkenyl, $C_1$-$C_{30}$ alkoxy, polyoxy($C_2$-$C_6$)alkylene, $C_1$-$C_{30}$ alkylamide, ($C_{12}$-$C_{22}$)alkylamido($C_2$-$C_6$)alkyl, ($C_{12}$-$C_{22}$)alkylacetate, $C_1$-$C_{30}$ hydroxyalkyl.

More preferably, at least one of the groups $R_1$ to $R_4$ comprises from 12 to 24 carbon atoms.

The aliphatic groups may comprise heteroatoms such as, in particular, oxygen, nitrogen, sulfur and halogens.

$X^-$ is an anionic counterion chosen from halides.

Among the quaternary ammonium salts of formula (I), preference is given firstly to tetraalkylammonium halides such as tetraalkylammonium chlorides, for instance tetraalkylammonium or alkyltrimethylammonium halides such as dialkyldimethylammonium or alkyltrimethylammonium chlorides in which the alkyl group contains from approximately 12 to 22 carbon atoms, in particular halides such as behenyltrimethylammonium chloride, distearyldimethylammonium chloride, cetyltrimethylammonium chloride, benzyldimethylstearylammonium chloride, hydroxyethyl oleyldimethylammonium chloride, or else, and palmitylamidopropyltrimethylammonium halide, such as the chloride or stearamidopropyldimethyl(myristyl acetate)ammonium chloride.

Mentions may be made of the products, for example, sold under the name Dehyquart A OR by Cognis (INCI name: Cetrimonium chloride), products sold under the name Chimexane CL by the company Chimex (INCI name: hydroxyl oleyldimonium chloride), products sold under the name Fentacare® 2232 EF by the company Rhodia (behenyltrimethylammonium chloride, with INCI name: behentrimonium chloride), or a mixture thereof.

More particularly, according to one embodiment of the invention, the cationic surfactant is present in an amount ranging from 0.01% to 10% by weight, preferably 0.05% to 5% by weight, relative to the total weight of the composition.

**B - Silicone-in-water (or oil-in-water) emulsion**

The cosmetic composition according to the invention further comprises an oil-in-water emulsion having $D_{50}$ particle size of less than 350 nm and that comprises:

- a silicone mixture comprising (i) a trialkylsilyl terminated dialkylpolysiloxane having a viscosity of from 40,000 to

less than 100,000 mPa.s at 25°C and (ii) an amino silicone having a viscosity of from 1,000 to 15,000 mPa.s at 25°C and an amine value of from 2 to 10 mg of KOH per gram of amino silicone;

- a mixture of emulsifiers comprising one or more nonionic emulsifiers, wherein the mixture of emulsifiers has a HLB value of from 10 to 16; and
- water.

In the oil-in-water emulsion, or silicone-in-water emulsion, one liquid phase (the dispersed phase) is dispersed in the other liquid phase (the continuous phase); in the present invention, the silicone mixture, or silicone phase, is dispersed in the continuous aqueous phase.

The silicone mixture comprises one or more trialkylsilyl terminated dialkylpolysiloxanes that are preferably of formula (II):

$R'_3SiO(R'_2SiO)_pSiR'_3$ formula (II)

wherein:

- R', same or different, is a monovalent hydrocarbon radical having from 1 to 18 carbon atoms, preferably from 1 to 6 carbon atoms, even better from 1 to 3 carbon atoms, more preferably methyl, and
- p is an integer of from 500 to 2000, preferably of from 1000 to 2000.

The trialkylsilyl terminated (or end-blocked or $\alpha,\omega$-position) dialkylpolysiloxanes according to the invention have a viscosity of from 40,000 to less than 100,000 mPa.s (100,000 excluded) at 25°C, preferably a viscosity of from 40,000 to 70,000 mPa.s at 25°C, more preferably a viscosity of from 51,000 to 70,000 mPa.s at 25°C.

The trialkylsilyl terminated dialkylpolysiloxanes according to the invention are preferably linear but may contain additionally to the $R'_2SiO_{2/2}$ units (D-units) in formula (II), $RSiO_{3/2}$ units (T-units) and/or $SiO_{4/2}$ units (Q-units), wherein R', same or different, is a monovalent hydrocarbon radical having from 1 to 18 carbon atoms.

Preferably, R', same or different, are alkyl radicals, preferably $C_1$-$C_{28}$ alkyl radicals, such as the methyl, ethyl, n-propyl, isopropyl, 1-n-butyl, 2-n-butyl, isobutyl, tert-butyl, n-pentyl, isopentyl, neopentyl and tert-pentyl radicals, hexyl radicals, such as the n-hexyl radical, heptyl radicals such as the n-heptyl radical, octyl radicals such as the n-octyl radical and isooctyl radicals, such as the 2,2,4-trimethylpentyl radical, nonyl radicals, such as the n-nonyl radicals, decyl radicals, such as the n-decyl radical, dodecyl radicals, such as the n-dodecyl radical, and octadecyl radicals, such as the n-octadecyl radical; alkenyl radicals such as the vinyl and ally radical; cycloalkyl radicals, such as the cyclopentyl, cyclohexyl, cycloheptyl and methylcyclohexyl radicals; aryl radicals, such as the phenyl, naphthyl, anthryl and phenanthryl radical; alkaryl radicals, such as the o-, m- and p-tolyl radicals, xylyl radicals and ethylphenyl radicals; and aralkyl radicals such as the benzyl radical and the a- and the b-phenylethyl radical. Most preferred is the methyl radical.

Preferably, the trialkylsilyl terminated dialkylpolysiloxanes are trimethylsilyl terminated PDMS (polydimethylsiloxanes or dimethicones).

The silicone mixture comprises one or more amino silicones, that are preferably of formula (III):

$XR_2Si(OSiAR)_n(OSiR_2)_mOSiR_2X$ formula (III)

wherein:

- R, same or different, is a monovalent hydrocarbon radical having from 1 to 18 carbon atoms, preferably from 1 to 6 carbon atoms, even better from 1 to 3 carbon atoms, more preferably methyl,
- X, same or different, is R or a hydroxyl (OH) or a $C_1$-$C_6$-alkoxy group; preferably X is R, i.e. a monovalent hydrocarbon radical having from 1 to 18 carbon atoms, preferably from 1 to 6 carbon atoms, even better from 1 to 3 carbon atoms, more preferably methyl, and
- A is an amino radical of the formula $-R^1-[NR^2-R^3-]_XNR^2_2$, or the protonated amino forms of said amino radical, wherein $R^1$ is a $C_1$-$C_6$-alkylene radical, preferably a radical of the formula - $CH_2CH_2CH_2$- or $-CH_2CH(CH_3)CH_2$-, $R^2$, same or different, is a hydrogen atom or a $C_1$-$C_4$-alkyl radical, preferably a hydrogen atom, $R^3$ is a $C_1$-$C_6$-alkylene radical, preferably a radical of the formula $-CH_2CH_2$-, and x is 0 or 1;
- m+n is an integer from 50 to about 1000, preferably from 50 to 600.

Preferably, A is an amino radical of the formula $-R^1-[NR^2-R^3-]_XNR^2_2$, or the protonated amino forms of said amino radical, wherein $R^1$ is $-CH_2CH_2CH_2$- or $-CH_2CH(CH_3)CH_2$-, $R^2$ are hydrogen atoms, $R^3$ is $-CH_2CH_2$-, and x is 1.

Preferably, R, same or different, are alkyl radicals, preferably $C_1$-$C_{28}$ alkyl radicals, such as the methyl, ethyl, n-propyl, isopropyl, 1-n-butyl, 2-n-butyl, isobutyl, tert-butyl, n-pentyl, isopentyl, neopentyl and tert-pentyl radicals, hexyl

radicals, such as the n-hexyl radical, heptyl radicals such as the n-heptyl radical, octyl radicals such as the n-octyl radical and isooctyl radicals, such as the 2,2,4-trimethylpentyl radical, nonyl radicals, such as the n-nonyl radicals, decyl radicals, such as the n-decyl radical, dodecyl radicals, such as the n-dodecyl radical, and octadecyl radicals, such as the n-octadecyl radical; alkenyl radicals such as the vinyl and ally radical; cycloalkyl radicals, such as the cyclopentyl, cyclohexyl, cycloheptyl and methylcyclohexyl radicals; aryl radicals, such as the phenyl, naphthyl, anthryl and phenanthryl radical; alkaryl radicals, such as the o-, m- and p-tolyl radicals, xylyl radicals and ethylphenyl radicals; and aralkyl radicals such as the benzyl radical and the a- and the b-phenylethyl radical. Most preferred is the methyl radical.

The amino silicones according to the invention have a viscosity of from 1,000 to 15,000 mPa.s at 25°C, preferably of from 1,500 to 15,000 mPa.s.

The amino silicones according to the invention have an amine value of from 2 to 10 mg of KOH per gram of amino silicone, preferably of from 3,5 to 8 mg.

The mole percent of amine functionality is preferably in the range of from about 0.3 to about 8%. Examples of amino silicones useful in the silicone mixture according to the invention include trialkylsilyl terminated amino silicone.

Most preferably, amino silicones are trimethylsilyl terminated aminoethylaminopropylmethylsiloxane, most preferably trimethylsilyl terminated aminoethylaminopropylmethylsiloxane - dimethylsiloxane copolymers.

The amino radical A can be protonated partially or fully by adding acids to the amino silicone, wherein the salt forms of the amino radical are obtained. Examples of acids are carboxylic acids with 3 to 18 carbon atoms which can be linear or branched, such as formic acid, acetic acid, propionic acid, butyric acid, pivalic acid, sorbic acid, benzoic acid, salicylic acid. The acids are preferably used in amounts of from 0.1 to 2.0 mol per 1 mol of amino radical A in the amino silicone of formula (III).

The silicone mixture preferably comprises (i) one or more trialkylsilyl terminated dialkylpolysiloxanes having a viscosity of from 40,000 to less than 100,000 mPa.s at 25°C in a quantity of from 70 to 90% by weight, preferably from 75 to 85% by weight and (ii) one or more amino silicones having a viscosity of from 1,000 to 15,000 mPa.s at 25°C and an amine value of from 2 to 10 mg of KOH per gram of amino silicone, in a quantity of from 10 to 30% by weight, preferably from 15 to 25% by weight, relative to the total weight of the silicone mixture.

The oil-in-water emulsion further comprises a mixture of emulsifiers that comprises one or more nonionic emulsifiers. It could optionally comprise one or more cationic surfactants.

The mixture of emulsifiers has a HLB value from 10 to 16.

The nonionic emulsifiers can be chosen among the nonionic surfactants previously described.

The nonionic emulsifiers could preferably be chosen among ethoxylated aliphatic alcohols, polyoxyethylene surfactants, carboxylic esters, polyethylene glycol esters, sorbitol ester and their ethoxylated derivatives, glycol esters of fatty acids, carboxylic amides, monoalkanolamine condensates, polyoxyethylene fatty acid amides.

Preferably, nonionic emulsifiers are selected from:

(i) polyoxyalkylene alkyl ethers, especially (poly)ethoxylated fatty alcohols of formula: $R_3\text{-}(OCH_2CH_2)_cOH$ with:

- $R_3$ representing a linear or branched $C_8$-$C_{40}$ alkyl or alkenyl group, preferably $C_8$-$C_{30}$ alkyl or alkenyl group, optionally substituted with one or more hydroxyl groups, and
- c being an integer between 1 and 200 inclusive, preferentially between 2 and 150 and more particularly between 4 and 50, most preferably between 8 and 20.
  The (poly)ethoxylated fatty alcohols are more particularly fatty alcohols comprising from 8 to 22 carbon atoms, oxyethylenated with 1 to 30 mol of ethylene oxide (1 to 30 OE);

(ii) polyoxyalkylene ($C_8$-$C_{32}$)alkylphenyl ethers,
(iii) polyoxyalkylene sorbitan ($C_8$-$C_{32}$) fatty acid esters, especially polyethoxylated fatty acid esters of sorbitan preferably containing from 2 to 40 ethylene oxide units, most preferably from 2 to 20 ethylene oxide units; preferably polyoxyethylenated sorbitan ($C_{10}$-$C_{24}$) fatty acid esters preferably containing from 2 to 40 ethylene oxide units, most preferably from 2 to 20 ethylene oxide units; and
(iv) polyoxyethylenated ($C_8$-$C_{32}$) fatty acid esters containing for example from 2 to 150 mol of ethylene oxide; preferably polyoxyethylenated ($C_{10}$-$C_{24}$) fatty acid esters containing for example from 2 to 150 mol of ethylene oxide.

Preferably, the nonionic emulsifiers could be selected from alkyl ether of polyalkyleneglycol and alkyl esters of polyalkyleneglycol; preferably of polyethyleneglycol.

Some useful emulsifiers are:

- polyethyleneglycol octyl ether; polyethyleneglycol lauryl ether; polyethyleneglycol tridecyl ether; polyethyleneglycol cetyl ether; polyethyleneglycol stearyl ether; among these, mention may be made more particularly of trideceth-3, trideceth-10 and steareth-6.

- polyethyleneglycol nonylphenyl ether; polyethyleneglycol dodecylphenyl ether; polyethyleneglycol cetylphenyl ether; polyethyleneglycol stearylphenyl ether;
- polyethyleneglycol sorbitan monostearate, polyethyleneglycol sorbitan monooleate.
- polyethyleneglycol stearate, and especially PEG 100 stearate.

Most preferably, the nonionic emulsifiers are chosen among steareth-6, PEG-100 stearate, trideceth-3 and trideceth-10 and their mixture; preferably, all these emulsifiers are present in the mixture of emulsifiers.

The mixture of emulsifiers could comprise one or more cationic emulsifiers that could be selected among tetraalkylammonium halides, tetraarylammonium halides, tetraalkylarylammonium halides, and their salts; quaternary ammonium compounds including salts; preferably, the cationic emulsifiers could be chosen among cetrimonium halides or behentrimonium halides, such as chloride.

The oil-in-water emulsion preferably comprises the mixture of emulsifiers in a total amount of from 5 to 15% by weight, preferably of from 8 to 15% by weight, most preferably of from 10 to 12% by weight, relative to the total weight of the emulsion.

The oil-in-water emulsion preferably comprises nonionic emulsifiers in a total amount of from 5 to 15% by weight, preferably of from 8 to 15% by weight, most preferably of from 10 to 12% by weight, relative to the total weight of the emulsion.

The oil-in-water emulsion preferably comprises cationic emulsifiers, when present, in a total amount of from 0,5 to 1,5% by weight, relative to the total weight of the emulsion.

The oil-in-water emulsion preferably comprises the silicone mixture in a total amount of from 40 to 60% by weight, preferably of from 45 to 55% by weight, relative to the total weight of the emulsion.

The oil-in-water emulsion preferably comprises the trialkylsilyl terminated dialkylpolysiloxane(s) in a total amount of from 35 to 45% by weight, preferably of from 38-42% by weight, relative to the total weight of the emulsion.

The oil-in-water emulsion preferably comprises the amino silicone(s) in a total amount of from 5 to 15% by weight, preferably of from 8-12% by weight, relative to the total weight of the emulsion. The oil-in-water emulsion comprises water preferably in an amount of from 25 to 50% by weight, preferably of from 30 to 45% by weight, most preferably of from 35 to 42% by weight, relative to the total weight of the emulsion.

A method of preparation of the oil-in-water emulsion preferably comprises:

- a step of mixing one or more trialkylsilyl terminated dialkylpolysiloxanes of viscosity of from 40,000 to less than 100,000 mPa.s at 25°C and one or more amino silicones of viscosity of from 1,000 to 15,000 mPa.s at 25°C and an amine value of from 2 to 10 mg of KOH per gram of amino silicone, at a temperature of from 15°C to 40°C, preferably at 25°C, to obtain a mixed silicone fluid, then
- a step of adding a mixture of emulsifiers comprising one or more nonionic emulsifiers, wherein the mixture of emulsifiers has a HLB value from 10 to 16, to the mixed silicone fluid to obtain a silicone-emulsifier-mixture, then
- a step of homogenizing the silicone-emulsifier-mixture followed by
- a step of adding, preferably step-wise, water, preferably demineralized water, to obtain an oil-in-water emulsion having $D_{50}$ particle size of less than 350 nm.

The preparation of the mixture of emulsifiers could be made by mixing one or more nonionic emulsifiers.

The pH of the oil-in-water emulsion after neutralization is preferably of from 4 to 6.

The oil-in-water emulsion has $D_{50}$ particle size of less than 350 nm, preferably of from 100 to 300 nm, more preferably from 150 to 250 nm, most preferably from 160 to 200 nm. It corresponds to the average hydrodynamic particle diameter. The $D_{50}$ particle size is expressed in volume. The $D_{50}$ particle size could be measured by using a device ZetaSizer from Malvern, UK, model Nano-ZS, which is based on the Photon Correlation Spectroscopy (PCS) method.

Particle size measurement

Emulsion particle size is measured by using a device ZetaSizer from Malvern, UK, model Nano-ZS which is based on the Photon Correlation Spectroscopy (PCS) method. The $D_{50}$ value of particle size (average hydrodynamic particle diameter) is measured, wherein the evaluating algorithm is "cumulants analysis".

Take 0.5 g of the emulsion sample in a 250 ml beaker, 100 ml of demineralized water is poured into it and then mixed properly to get the sample test solution. The sample test solution is poured in the cuvette cell and is put into the slot of the instrument to measure the particle size of the emulsion. $D_{50}$ is defined as the value of the particle diameter at 50% in the cumulative distribution. For example, if $D_{50}$=170 nm, then 50% of the particles in the sample are larger than 170 nm, and 50% smaller than 170 nm or about 50% by volume of all droplets in said emulsion is 170 nm.

Viscosity measurement

The viscosity, especially of the silicones or of the emulsion, is measured at 25°C.

For viscosities between 1000 to 40,000 mPa.s at 25°C: the viscosity could be measured with an Anton Paar Rheometer; model MCR101, geometry single gap cylinder: CC27 spindle and shear rate of 1 $s^{-1}$ for 2 minutes, at 25°C.

For viscosities between 40,000 to 100,000 mPa.s at 25°C: the viscosity could be measured with an Anton Paar Rheometer; model MCR101, 25-6 cone (Cone-plate geometry: 25 mm dia. / 6° cone); the "Zero gap" setting being made and with a shear rate of 1 $s^{-1}$ for 2 minutes, at 25°C.

Three measurements are made for each sample and the viscosity value is taken at 60 secs. MCR Rheometer Series products work as per USP (US Pharmacopeia Convention) 912 - Rotational Rheometer methods.

Amine value measurement

The amine value is determined by acid-base titration using a potentiometer [Make: Veego; Model: VPT-MG]. 0.6 g of sample is taken in a 500 ml beaker and a toluene-butanol 1:1 mixture is added and stirred to mix the sample thoroughly; then the sample solution is titrated with a 0.1(N) HCl solution. A determination of the blank value with the toluene-butanol 1:1 mixture is also done. The calculation of the amine value is done by the above mentioned potentiometer.

The amine value is calculated according to the formula:

$$56.11 \times (V - V_{Blank}) \times N / W \text{ mg KOH/ g of sample,}$$

where V= Volume of HCl required in ml, $V_{Blank}$= Volume of HCl for blank value (without sample) with the toluene-butanol 1:1 mixture in ml; N= Normality of HCl, i.e. 0.1 N, W= weight of the sample taken in gram.

HLB Value

The term HLB is well known to those skilled in the art, and denotes the hydrophilic-lipophilic balance of a surfactant or emulsifier. In the present invention, HLB values refer to the values at 25°C. The HLB can be measured by experimental determination or can be calculated.

Calculation of HLB value of nonionic surfactants is calculated according to the equation : HLB = (E + P)/5, with E being the weight percentage of oxyethylene content and P being the weight percentage of polyhydric alcohol content, described in to the publication Griffin, J. Soc. Cosm. Chem. 1954 (vol.5, n°4), pp.249-256.

It can also experimentally be determined according to the book of F. Puisieux and M. Seiller, entitled "Galenica 5: Les systèmes disperses - Tome I - Agents de surface et émulsions - Chapitre IV - Notions de HLB et de HLB critique, pp. 153-194 - paragraph 1.1.2. Determination de HLB par voie experimentale [Experimental determination of HLB], pp.164-180".

The calculated HLB is the preferred HLB values that should be taken into account.

Said calculated HLB could be defined as being the following:

"calculated HLB = 20 × molar mass of the hydrophilic part/total molar mass."

For an oxyethylenated fatty alcohol, the hydrophilic part corresponds to the oxyethylene units condensed onto the fatty alcohol and the "calculated HLB" then corresponds to the "Griffin HLB" as defined hereabove.

For an ester or an amide, the hydrophilic part is naturally defined as being beyond the carbonyl group, starting from the fatty chain(s).

For ionic surfactants/emulsifiers, the HLB value of individual surfactant/emulsifier can be calculated applying the Davies formula as described in Davies JT (1957), "A quantitative kinetic theory of emulsion type, I. Physical chemistry of the emulsifying agent", Gas/Liquid and Liquid/Liquid Interface (Proceedings of the International Congress of Surface Activity): 426-438.

According to the formula, the HLB is derived by summing the hydrophilic/hydrophobic contribution afforded by the structural components of the emulsifier: HLB = (hydrophilic groups numbers) - n(group number per CH2 group) +7.

Approximate HLB values for some cationic emulsifiers are given in Table IV, in "Cationic emulsifiers in cosmetics", GODFREY, J. Soc. Cosmetic Chemists (1966) 17, pp 17-27.

When two emulsifiers A and B of known HLB are blended for use, the $HLB_{Mix}$ is said to be the required HLB for the mixture. This is expressed by the equation $(W_A HLB_A + W_B HLB_B)/ (W_A + W_B) = HLB_{Mix}$, where $W_A$ = the amount

(weight) of the first emulsifier (A) used, and $W_B$ = the amount (weight) of the second emulsifier (B); $HLB_A$, $HLB_B$ = the assigned HLB values for emulsifiers A and B; $HLB_{Mix}$ = the HLB of the mixture.

Advantageously, the composition according to the invention may comprise the oil-in-water emulsion in a quantity ranging of from 0.1% to 15% by weight, preferably from 0,5% to 10% by weight, more preferably from 1% to 8% by weight, even more preferably from 1,5% to 5% by weight, with respect to the total weight of the composition.

**C - Fatty alcohol(s)**

The composition of the present invention comprises at least one fatty alcohol.

The term "fatty alcohol" used herein refers to the compounds of formula R-OH (formula (IV)), which are linear or branched, saturated or unsaturated, and may contain from 8 to 40 carbon atoms. Optionally, R maybe substituted with two or more hydroxyl groups.

Optionally, the fatty alcohol may be oxyalkylenated or glycerolated.

More preferably, in the fatty alcohol of formula (IV), R denotes from 8 to 30 carbon atoms; R preferably denotes a $C_{12}$-$C_{24}$ alkyl or a $C_{12}$-$C_{24}$ alkenyl group. Optionally, R may be substituted with one or more hydroxyl groups and especially with one or two hydroxyl groups.

Examples that may be mentioned include cetyl alcohol, stearyl alcohol, behenyl alcohol, isocetyl alcohol, isostearyl alcohol, isobehenyl alcohol and oleyl alcohol, and mixtures thereof.

The alcohol is preferably chosen from cetyl alcohol, stearyl alcohol, behenyl alcohol, or mixtures thereof.

Examples of the fatty alcohols may be mentioned are, for instance, cetyl alcohol that is sold under the name Lanette® 16 by the company BASF; stearyl alcohol that is sold under the name Lanette® 18 by the company BASF; behenyl alcohol that is sold under the name Lanette® 22 by the company BASF; the mixture of cetyl alcohol and stearyl alcohol, which is sold under the name Lanette® O OR by the company BASF.

The fatty alcohol(s) may be present in the composition in a content ranging from 1% to 15%, preferably from 2% to 10% and more preferably from 3% to 8% by weight relative to the total weight of the composition.

**D - Additional silicone(s)**

The composition according to the present invention preferably comprises one or more additional silicones, which is different from the silicone-in-water (or oil-in-water) emulsion as described above. Such silicones can be chosen in particular from non-amino silicones, amino silicones and mixtures thereof.

In the present invention, the term "silicone" is intended to denote, in accordance with what is generally accepted, any organosilicon polymer or oligomer of linear or cyclic, branched or crosslinked structure, of variable molecular weight, obtained by polymerization and/or polycondensation of suitably functionalized silanes, and consisting essentially of a repetition of main units in which the silicon atoms are linked together via oxygen atoms (siloxane bond -Si-O-Si-), optionally substituted hydrocarbon-based radicals being directly linked via a carbon atom to the said silicon atoms. The hydrocarbon-based radicals that are the most common are alkyl radicals, especially $C_1$-$C_{10}$ alkyl radicals, and in particular methyl, fluoroalkyl radicals, the alkyl part of which is $C_1$-$C_{10}$, and aryl radicals and in particular phenyl.

According to the present invention, the term "non-amino silicone" denotes any silicone not containing at least one primary, secondary or tertiary amine, or a quaternary ammonium group.

The non-amino silicones, which can be used in the composition according to the invention, are, in particular, polyorganosiloxanes that may be in the form of oils, waxes, resins or gums.

For the purposes of the present invention, the term "amino silicone" means any silicone comprising at least one primary, secondary or tertiary amine function or a quaternary ammonium group. Preferably, the additional silicone that is suitable in the present invention is chosen from non-amino silicones.

Organopolysiloxanes are defined in greater detail in Walter Noll's Chemistry and Technology of Silicones (1968), Academic Press.

The silicones may be volatile or non-volatile. Preferably, the silicones are non-volatile silicones.

When the silicones are non-volatile, use is preferably made of polyalkylsiloxanes, polyarylsiloxanes, polyalkylarylsiloxanes, silicone gums and resins, and polyorganosiloxanes optionally modified with organofunctional groups, and mixtures thereof.

These silicones are more particularly chosen from polyalkylsiloxanes, among which mention may be made mainly of polydimethylsiloxanes containing trimethylsilyl end groups (Dimethicone according to the CTFA name) having a viscosity of from $5\times10^{-6}$ to 2.5 $m^2/s$ at 25°C and preferably $1\times10^{-5}$ to 1 $m^2/s$. The viscosity of the silicones is measured, for example, at 25°C according to standard ASTM 445 Appendix C.

Among these polyalkylsiloxanes, mention may be made, in a non-limiting manner, of the following commercial products:

- the Silbione oils of the 47 and 70 047 series or the Mirasil oils sold by the company Rhodia, for instance the oil 70 047 V 500 000,
- the oils of the Mirasil series sold by the company Rhodia, in particular Mirasil® DM 500 000 sold by Rhodia.
- the oils of the 200 series from the company Dow Coming, such as, more particularly, DC200 with a viscosity of 60 000 mPas (cSt),
- the Viscasil oils from the company General Electric and certain oils of the SF series (SF 96, SF 18) from the company General Electric,

The polyalkylsiloxanes may also be in form of a silicone in water emulsion. Such products are available on the market, for example, emulsion of organopolysiloxane which contains 75% of dimethicone, sold by the company Shin-etsu under the name X52-2127.
Mention may also be made of polydimethylsiloxanes containing dimethylsilanol end groups (Dimethiconol according to the CTFA name) such as the oils of the 48 series from the company Rhodia.
Mention may also be made of polydimethylsiloxanes containing $\alpha,\omega$-silanol groups.
In this category of polyalkylsiloxanes, mention may also be made of the products sold under the names Abil Wax 9800 and 9801 by the company Goldschmidt, which are poly($C_1$-$C_{20}$)alkylsiloxanes.
The polyalkylarylsiloxanes are particularly chosen from linear and/or branched polydimethylmethylphenylsiloxanes and polydimethyldiphenylsiloxanes with a viscosity of from $1\times10^{-5}$ to $5\times10^{-2} m^2/s$ at 25°C.
Among these polyalkylarylsiloxanes, examples that may be mentioned include the products sold under the following names:

- Silbione oils of the 70 641 series from the company Rhodia,
- the oils of the Rhodorsil 70 633 and 763 series from the company Rhodia,
- the oil Dow Corning 556 Cosmetic Grade Fluid from the company Dow Coming,
- silicones of the PK series from the company Bayer, such as the product PK20,
- the silicones of the PN and PH series from the company Bayer, such as the products PN1000 and PH1000,
- certain oils of the SF series from the company General Electric, such as SF 1023, SF 1154, SF 1250 and SF 1265.

The silicone gums that may be present in the composition according to the invention are especially polydiorganosiloxanes having high number-average molecular masses of between 200 000 and 1 000 000, preferably between 500 000 and 1 000 000, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.
Mention may be made more particularly of the following products:

- polydimethylsiloxane gums,
- polydimethylsiloxane/methylvinylsiloxane gums,
- polydimethylsiloxane/diphenylsiloxane gums,
- polydimethylsiloxane/phenylmethylsiloxane gums,
- polydimethylsiloxane/diphenylsiloxane/methylvinylsiloxane gums.

Products that may be used are the following:

- polydimethylsiloxane sold by the company Bluestar under the name Mirasil DM 500 000, or by the company Dow Coming under the name Xiameter PMX-200 Silicone fluid (500 000 mPas (cSt)),
- mixtures formed from a polydimethylsiloxane hydroxylated at the end of the chain (known as dimethiconol according to the nomenclature of the CTFA dictionary) and from a cyclic polydimethylsiloxane (known as cyclomethicone according to the nomenclature of the CTFA dictionary), such as the product Q2 1401 sold by the company Dow Coming,
- mixtures formed from a polydimethylsiloxane gum with a cyclic silicone, such as the product SF 1214 Silicone Fluid from the company General Electric, this product being an SF 30 gum corresponding to a dimethicone, having a number-average molecular weight of 500 000, dissolved in the oil SF 1202 Silicone Fluid corresponding to decamethylcyclopentasiloxane,
- mixtures of two PDMSs with different viscosities, and more particularly of a PDMS gum and a PDMS oil, such as the product SF 1236 from the company General Electric. The product SF 1236 is a mixture of a gum SE 30 defined above, with a viscosity of 20 $m^2/s$ and of oil SF 96 with a viscosity of $5\times10^{-6} m^2/s$. This product preferably comprises 15% of gum SE 30 and 85% of oil SF 96.

According to an embodiment, when exists, the additional silicone is present in an amount ranging from 0.01% to 10% by weight, preferably from 0.05% to 5% by weight, relative to the total weight of the composition.

**Others ingredients**

The composition of the present invention may comprise water, for example in a quantity of from 40 to 99% by weight, preferably from 50 to 98% by weight, most preferably from 55 to 95% by weight, relative to the total weight of the composition.

The composition according to the present invention may further comprise one or more additive(s) other than the compounds of the invention.

As additives that may be used in accordance with the invention, mention may be made of fatty substances, anionic, nonionic or amphoteric polymers or mixtures thereof, cationic surfactants, antidandruff agents, anti-seborrhoea agents, agents for preventing hair loss and/or for promoting hair regrowth, vitamins and provitamins including panthenol, sunscreens, mineral or organic pigments, sequestrants, plasticizers, solubilizers, acidifying agents, mineral or organic thickeners, especially polymeric thickeners, opacifiers, pearlescent or nacreous agents, antioxidants, hydroxyacids, fragrances and preserving agents.

According to a preferred embodiment, the present invention relates to a composition for conditioning hair, comprising, relative to the total weight of the composition:

(i) from 0.05% to 5% by weight of behenyltrimethylammonium chloride;
(ii) from 1,5% to 5% by weight of an oil-in-water emulsion having $D_{50}$ particle size of less than 350 nm and comprising :

- the mixture of emulsifiers in a total amount of from 5 to 15% by weight, preferably of from 8 to 15% by weight, most preferably of from 10 to 12% by weight, relative to the total weight of the emulsion; and/or
- nonionic emulsifiers in a total amount of from 5 to 15% by weight, preferably of from 8 to 15% by weight, most preferably of from 10 to 12% by weight, relative to the total weight of the emulsion; and/or
- cationic emulsifiers in a total amount of from 0,5 to 1,5% by weight, relative to the total weight of the emulsion; and/or
- the silicone mixture in a total amount of from 40 to 60% by weight, preferably of from 45 to 55% by weight, relative to the total weight of the emulsion; and/or
- the trialkylsilyl terminated dialkylpolysiloxane(s) in a total amount of from 35 to 45% by weight, preferably of from 38-42% by weight, relative to the total weight of the emulsion; and/or
- the amino silicone(s) in a total amount of from 5 to 15% by weight, preferably of from 8-12% by weight, relative to the total weight of the emulsion; and/or
- water in an amount of from 25 to 50% by weight, preferably of from 30 to 45% by weight, most preferably of from 35 to 42% by weight, relative to the total weight of the emulsion; and

(iii) from 3% to 8% by weight of at least one fatty alcohol selected from the group consisting of cetyl alcohol, stearyl alcohol, or a mixture thereof.

Needless to say, a person skilled in the art will take care to select these optional additives such that the advantageous properties intrinsically associated with the composition according to the invention are not, or are not substantially, adversely affected by the envisaged addition(s).

The composition according to the invention may take the form of thickened liquid, creams or gel, They may also take the form of lotions.

The composition according to the invention is a hair composition; preferably it is a hair conditioning composition.

Another subject of the present invention is a process for the cosmetic treatment of hair, preferably for conditioning hair, comprising the steps of applying to the hair, preferably in a wet state,
the composition described above, and optionally rinsing them with water after an optional leave-on time.

The leave-on time of the composition on hair may range from a few seconds to 15 minutes, better still from 5 seconds to 10 minutes and even better still from 10 seconds to 5 minutes.

The composition may be applied to wet or dry hair.

The examples that follow serve to illustrate the invention without, however, being limiting in nature.

The stability of the emulsion is determined after 3 months of storage at 45°C; the stability is determined by no change in property of the emulsion; if the property changes, or if the oil and the aqueous phases separate, the emulsion is said to be unstable.

## Example 1: Preparation of the oil-in-water emulsion

Transfer 450 g of amino silicone fluid (trimethylsilyl terminated aminoethylaminopropylmethylsiloxane - dimethylsiloxane copolymer with amine value of 7,2 mg of KOH/gm sample, and a viscosity of 5600 mPa.s at 25°C) in emulsion tank. Start stirring and under stirring condition transfer 1800 g of trimethylsilyl terminated dimethylsiloxane polymer fluid of viscosity 61,500 mPa.s at 25°C in the same tank. Mix both the fluids for 2 hours at room temperature.

In a separate tank, transfer 49 g of steareth-6, 62 g of PEG100 stearate and heat to 60°C. Maintain the temperature till both emulsifiers become liquid. Then add 31 g of trideceth-3 and 350 g of trideceth-10 (80% of active matter). This nonionic emulsifiers mixture has an HLB value = 11.25. Then add 80 g water and 6.2 g glacial acetic acid in the tank and start mixing. Continue mixing till whole mass become a creamy paste. Whole paste is transfer to emulsion tank. Homogenize for 30 minutes at room temperature. Add 79.6 g demineralized water and homogenize for 60 minutes. Add 72.7 g demineralized water and homogenize for 50 minutes. Add 197.4 g demineralized water and homogenize for 5 minutes. Add 294.3 g demineralized water and homogenize for 5 minutes. Add 180 g demineralized water and homogenize for 5 minutes. Add 180 g demineralized water and homogenize for 5 minutes. Add 197.4 g demineralized water and homogenize for 5 minutes. Add 197.4 g demineralized water and homogenize for 3 minutes. Add 228.5 g demineralized water and homogenize for 3 minutes. Lastly add 40.5 g 2-phenoxyethanol as a biocide and homogenize for 3 minutes.

An stable oil-in-water emulsion having $D_{50}$ particle size of 170 nm is obtained.

## Example 2: Hair formulas

The following formulas were prepared from the ingredients indicated in the table below, in which the amounts are given as mass percentages of active material relative to the total weight of the total amount of the formulas.

| Ingredient | Invention formula | | | | Comparative formula | |
|---|---|---|---|---|---|---|
| | % by weight by active material | | | | | |
| | **1** | **2** | **3** | **4** | **3'** | **2'** |
| Behentrimonium chloride (Fentacare® 2232 EF from Rhodia) | 3.2 | 3.2 | 3.2 | 3.2 | 3.2 | 3.2 |
| Cetearyl alcohol (Lanette® O OR from BASF) | 5.5 | 5.5 | 5.5 | 7.5 | 5.5 | 0 |
| Cetyl Ester (Miraceti from Laserson) | 0.8 | 0.8 | 0.8 | 0.8 | 0.8 | 0.8 |
| Oil-in-water emulsion from example 1 | 0.6 | 1.2 | 1.2 | 3 | 0 | 1.2 |
| Dimethicone (X52-2127 from Shin-etsu, which contains 75% of dimethicone, with a viscosity of 2000 to 10000 mPa·s) | 1.2 | 1.2 | 0 | 0 | 0 | 1.2 |
| Dimethicone (Mirasil® DM 500 000 by Bluestar) | 0.6 | 0.6 | 0 | 0 | 0 | 0.6 |
| Water | QS 100 | QS 100 | QS 100 | QS 100 | QS 100 | QS 100 |
| Fragrance | 0.5 | 0.5 | 0.5 | 0.5 | 0.5 | 0.5 |

Comparative formula **3'** (corresponding to the invention formula **3**) does not contain the (ii) oil in water emulsion as claimed; comparative formula **2'** (corresponding to the invention formula **2**) does not contain (iii) fatty alcohol as claimed.

The above mentioned formulas were prepared following a conventionally known formulation process for hair conditioning compositions.

## Example 3: evaluation example

The invention and comparative formulas were applied on hair swatches and the coefficient of friction of the hair swatches were evaluated using the analyzing tool Coefficient of Friction MTT175 produced by the company Dia-Stron. The lower the coefficient of friction is, the better the cosmetic property of the formula.

The result is as follow:

| Coefficient of Friction | Invention formulas | | | | Comparative formulas | |
|---|---|---|---|---|---|---|
| | **1** | **2** | **3** | **4** | **3'** | **2'** |
| | 0.25 | 0.257 | 0.357 | 0.344 | 0.563 | Emulsion cannot be prepared |

According to the result obtained above, the invention formulas **1** to **4** present improved cosmetic properties and therefore affording improved hair softness, smoothness and disentangling, comparing to the comparative formula **3'.**

Besides, the comparative formula **2'** is not a stable emulsion, whereas the invention formulas **1** to **4** are stable over time, i.e., stored for 2 months at temperature between 4 °C and 45°C.

Lastly, it is observed that the invention formulas **1** and **2** are the preferred formulas according to the invention.

## Claims

**1.** A hair composition comprising:

(i) at least one cationic surfactant of the formula (1),

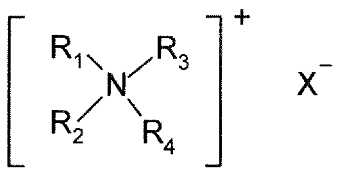

(1)

wherein:

$R_1$ to $R_4$, identical or different, represent a linear or branched aliphatic group comprising from 1 to 30 carbon atoms, an aromatic group such as aryl or alkylaryl,
at least one of the groups $R_1$ to $R_4$ comprising from 8 to 30 carbon atoms, and
$X^-$ is an anionic counterion chosen from halides;

(ii) an oil-in-water emulsion having $D_{50}$ particle size of less than 350 nm, expressed in volume and measured with the Photon Correlation Spectroscopy (PCS) method, and comprising:

- a silicone mixture comprising (i) a trialkylsilyl terminated dialkylpolysiloxane having a viscosity of from 40,000 to less than 100,000 mPa.s at 25°C at a shear rate of 1 $s^{-1}$, and (ii) an amino-silicone having a viscosity of from 1,000 to 15,000 mPa.s at 25°C at a shear rate of 1 $s^{-1}$, and an amine value of from 2 to 10 mg of KOH per gram of amino-silicone,
the viscosities being determined with the US Pharmacopeia Convention (USP) 912 rotational rheometer methods;
- a mixture of emulsifiers comprising one or more nonionic emulsifiers, wherein the mixture of emulsifiers has a HLB value from 10 to 16; and
- water; and

(iii) at least one $C_{8-40}$ fatty alcohol.

**2.** The composition of claim 1, wherein in the formula (I), at least one of the groups $R_1$ to $R_4$ comprises from 12 to 24 carbon atoms, wherein the aliphatic groups may comprise heteroatoms, preferably oxygen, nitrogen, sulfur or halogens; preferably the surfactant of formula (1) is selected from the group consisting of tetraalkylammoniumhalides halides in which the alkyl group contains from 12 to 22 carbon atoms, or a mixture thereof; more preferably selected from the group consisting of behenyltrimethylammonium chloride, distearyldimethylammonium chloride, cetyltrimethylammonium chloride, benzyldimethylstearylammonium chloride, hydroxyethyl oleyldimethylammonium chloride, palmitylamidopropyltrimethylammonium chloride, stearamidopropyldimethyl(myristyl acetate)ammonium chloride, or a mixture thereof; even more preferably the surfactant of formula (I) is behenyltrimethylammonium chloride.

**3.** The composition of claim 1 or 2, wherein the surfactant of formula (I) presents in an amount ranging from 0.01% to 10% by weight, preferably 0.05% to 5% by weight, relative to the total weight of the composition.

**4.** The composition according to any one of the preceding claims 1 to 3, wherein the trialkylsilyl terminated dialkylpolysiloxanes are of formula (II):

$$R'_3SiO(R'_2SiO)_pSiR'_3 \qquad \text{formula (II)}$$

wherein:

- R', same or different, is a monovalent hydrocarbon radical having from 1 to 18 carbon atoms, from 1 to 6 carbon atoms, and
- p is an integer of from 500 to 2000, preferably of from 1000 to 2000;

and preferably are PDMS (polydimethylsiloxanes or dimethicones).

**5.** The composition according to any one of the preceding claims 1 to 4, wherein the trialkylsilyl terminated dialkylpolysiloxanes have a viscosity of from 40,000 to 70,000 mPa.s at 25°C, more preferably a viscosity of from 51,000 to 70,000 mPa.s at 25°C.

**6.** The composition according to any one of the preceding claims 1 to 5, wherein the amino silicones are of formula (III):

$$XR_2Si(OSiAR)_n(OSiR_2)_mOSiR_2X \qquad \text{formula (III)}$$

wherein:

- R, same or different, is a monovalent hydrocarbon radical having from 1 to 18 carbon atoms, preferably from 1 to 6 carbon atoms,
- X, same or different, is R or a hydroxyl (OH) or a $C_1$-$C_6$-alkoxy group; preferably X is R;
- A is an amino radical of the formula $-R^1-[NR^2-R^3-]_XNR^2_2$, or the protonated amino forms of said amino radical, wherein $R^1$ is a $C_1$-$C_6$-alkylene radical, preferably a radical of the formula - $CH_2CH_2CH_2$- or $-CH_2CH(CH_3)CH_2-$, $R^2$, same or different, is a hydrogen atom or a $C_1$-$C_4$-alkyl radical, preferably a hydrogen atom, $R^3$ is a $C_1$-$C_6$-alkylene radical, preferably a radical of the formula
- $CH_2CH_2$-, and a is 0 or 1;
- m+n is an integer from 50 to about 1000, preferably from 50 to 600;

preferably, A being an amino radical of the formula $-R^1-[NR^2-R^3-]_XNR^2_2$, or the protonated amino forms of said amino radical, wherein R' is $-CH_2CH_2CH_2-$ or $-CH_2CH(CH_3)CH_2-$, $R^2$ are hydrogen atoms, $R^3$ is $-CH_2CH_2-$, and x is 1.

**7.** The composition according to any one of the preceding claims 1 to 6, wherein the amino silicones have a viscosity of from 1,500 to 15,000 mPa.s; and/or an amine value of from 3,5 to 8 mg of KOH per gram of amino silicone.

**8.** The composition according to any one of the preceding claims 1 to 7, wherein the silicone mixture comprises (i) one or more trialkylsilyl terminated dialkylpolysiloxanes having a viscosity of from 40,000 to less than 100,000 mPa.s at 25°C in a quantity of from 70 to 90% by weight, preferably from 75 to 85% by weight and (ii) one or more amino silicones having a viscosity of from 1,000 to 15,000 mPa.s at 25°C and an amine value of from 2 to 10 mg of KOH per gram of amino silicone, in a quantity of from 10 to 30% by weight, preferably from 15 to 25% by weight, relative to the total weight of the silicone mixture.

**9.** The composition according to any one of the preceding claims 1 to 8, wherein the mixture of emulsifiers comprises one or more emulsifiers chosen between:

(i) polyoxyalkylene alkyl ethers, especially (poly)ethoxylated fatty alcohols of formula: $R_3-(OCH_2CH_2)_cOH$ with:

- $R_3$ representing a linear or branched $C_8$-$C_{40}$ alkyl or alkenyl group, preferably $C_8$-$C_{30}$ alkyl or alkenyl group, optionally substituted with one or more hydroxyl groups, and
- c being an integer between 1 and 200 inclusive, preferentially between 2 and 150 and more particularly between 4 and 50, most preferably between 8 and 20,

the (poly)ethoxylated fatty alcohols are more particularly fatty alcohols comprising from 8 to 22 carbon atoms, oxyethylenated with 1 to 30 mol of ethylene oxide (1 to 30 OE);

(ii) polyoxyalkylene ($C_8$-$C_{32}$)alkylphenyl ethers,
(iii) polyoxyalkylene sorbitan ($C_8$-$C_{32}$) fatty acid esters, especially polyethoxylated fatty acid esters of sorbitan preferably containing from 2 to 40 ethylene oxide units, most preferably from 2 to 20 ethylene oxide units; preferably polyoxyethylenated sorbitan ($C_{10}$-$C_{24}$) fatty acid esters preferably containing from 2 to 40 ethylene oxide units, most preferably from 2 to 20 ethylene oxide units; and
(iv) polyoxyethylenated ($C_8$-$C_{32}$) fatty acid esters containing for example from 2 to 150 mol of ethylene oxide; preferably polyoxyethylenated ($C_{10}$-$C_{24}$) fatty acid esters containing for example from 2 to 150 mol of ethylene oxide.

**10.** The composition according to any one of the preceding claims 1 to 9, wherein the oil-in-water emulsion comprises:

- the mixture of emulsifiers in a total amount of from 5 to 15% by weight, preferably of from 8 to 15% by weight, most preferably of from 10 to 12% by weight, relative to the total weight of the emulsion; and/or
- nonionic emulsifiers in a total amount of from 5 to 15% by weight, preferably of from 8 to 15% by weight, most preferably of from 10 to 12% by weight, relative to the total weight of the emulsion; and/or
- cationic emulsifiers in a total amount of from 0.5 to 1,5% by weight, relative to the total weight of the emulsion; and/or
- the silicone mixture in a total amount of from 40 to 60% by weight, preferably of from 45 to 55% by weight, relative to the total weight of the emulsion; and/or
- the trialkylsilyl terminated dialkylpolysiloxane(s) in a total amount of from 35 to 45% by weight, preferably of from 38-42% by weight, relative to the total weight of the emulsion; and/or
- the amino silicone(s) in a total amount of from 5 to 15% by weight, preferably of from 8-12% by weight, relative to the total weight of the emulsion; and/or
- water in an amount of from 25 to 50% by weight, preferably of from 30 to 45% by weight, most preferably of from 35 to 42% by weight, relative to the total weight of the emulsion.

**11.** The composition according to any one of the preceding claims 1 to 10, wherein the oil-in-water emulsion (ii) having a $D_{50}$ particle size of from 100 to 300 nm, more preferably from 150 to 250 nm, most preferably from 160 to 200 nm, expressed in volume.

**12.** The composition according to any one of the preceding claims 1 to 11, wherein the oil-in-water emulsion (ii) presents in a quantity ranging of from 0.1% to 15% by weight, preferably from 0,5% to 10% by weight, more preferably from 1% to 8% by weight, even more preferably from 1,5% to 5% by weight, with respect to the total weight of the composition.

**13.** The composition according to any one of the preceding claims 1 to 12, wherein the fatty alcohol is selected from the compound of formula (IV):

R-OH formula (IV)

wherein:

R denotes from 8 to 30 carbon atoms, preferably a $C_{12}$-$C_{24}$ alkyl or a $C_{12}$-$C_{24}$ alkenyl group, wherein R is optionally substituted with one or more hydroxyl groups, preferably with one or two hydroxyl groups; more preferably, the fatty alcohol is selected from the group consisting of cetyl alcohol,
stearyl alcohol, behenyl alcohol, isocetyl alcohol, isostearyl alcohol, isobehenyl alcohol, oleyl alcohol, and mixtures thereof; even more preferably selected from the group consisting of cetyl alcohol, stearyl alcohol, or a mixture thereof.

**14.** The composition according to any one of the preceding claims 1 to 13, wherein the fatty alcohol presents in an amount ranging from 1% to 15%, preferably from 2% to 10% and more preferably from 3% to 8% by weight relative to the total weight of the composition.

**15.** The composition according to any one of the preceding claims 1 to 14, further comprising at least one additional silicone, chosen from polyorganosiloxanes in the form of oils, waxes, resins or gums; preferably selected from

polydiorganosiloxanes having high number-average molecular masses of between 200 000 and 1 000 000, preferably between 500 000 and 1 000 000, used alone or as a mixture in a solvent.

**16.** The composition of any one of the preceding claims 1 to 15, wherein the additional silicone presents in an amount ranging from 0.01% to 10% by weight, preferably from 0.05% to 5% by weight, relative to the total weight of the composition.

**17.** Process for the cosmetic treatment of hair, preferably for conditioning hair, comprising the steps of applying to the hair, preferably in a wet state, the composition according to any one of claims 1 to 16, and optionally rinsing them with water after an optional leave-on time.

**Patentansprüche**

**1.** Haarpflegezusammensetzung, umfassend:

(i) mindestens ein kationisches Tensid der Formel (I),

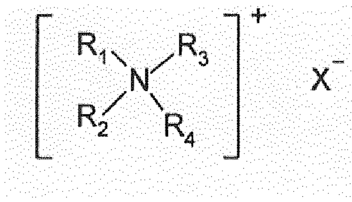

(I)

worin:

$R_1$ bis $R_4$, identisch oder verschieden, eine lineare oder verzweige aliphatische Gruppe mit 1 bis 30 Kohlenstoffatomen, eine aromatische Gruppe wie zum Beispiel Aryl oder Alkylaryl repräsentieren,
wobei mindestens eine der Gruppen $R_1$ bis $R_4$ von 8 bis 30 Kohlenstoffatome umfasst, und $X^-$ ein anionisches Gegenion ist, das aus Halogeniden ausgewählt ist;

(ii) eine Öl-in-Wasser-Emulsion mit einer Teilchengröße $D_{50}$ kleiner als 350 nm, ausgedrückt in Volumen und gemessen mit dem Verfahren der Photonenkorrelationsspektroskopie (PCS), und umfassend:

- eine Silikonmischung bestehend aus (i) einem trialkylsilylterminierten Dialkylpolysiloxan mit einer Viskosität von 40.000 bis weniger als 100.000 mPa.s bei 25°C bei einer Schergeschwindigkeit von 1 $s^{-1}$, und (ii) einem Aminosilikon mit einer Viskosität von 1.000 bis 15.000 mPa.s bei 25°C bei einer Schergeschwindigkeit von 1 $s^{-1}$, und mit einem Aminwert von 2 bis 10 mg KOH pro Gramm Aminosilikon,
wobei die Viskosität jeweils mit dem Rotationsrheometerverfahren 912 der US Pharmacopeia Convention (USP) ermittelt wurde;
- eine Mischung von Emulgatoren mit einem oder mehreren nichtionischen Emulgatoren, wobei die Mischung von Emulgatoren einen HLB-Wert von 10 bis 16 hat; und
- Wasser; und

(iii) mindestens einen $C_{8-40}$-Fettalkohol.

**2.** Zusammensetzung nach Anspruch 1, wobei in Formel (I) mindestens eine der Gruppen $R_1$ bis $R_4$ von 12 bis 24 Kohlenstoffatome umfasst, wobei die aliphatischen Gruppen Heteroatome, vorzugsweise Sauerstoff, Stickstoff, Schwefel oder Halogene umfassen können; vorzugsweise ist das Tensid von Formel (I) aus der aus Tetraalkylammoniumhalogeniden bestehenden Gruppe ausgewählt, bei der die Alkylgruppe von 12 bis 22 Kohlenstoffatome umfasst, oder eine Mischung davon; mehr bevorzugt ausgewählt aus der aus Behenyltrimethylammoniumchlorid, Distearyldimethylammoniumchlorid, Cetyltrimethylammoniumchlorid, Benzyldimethylstearylammoniumchlorid, Hydroxyethyloleyldimethylammoniumchlorid, Palmitylamidopropyltrimethylammoniumchlorid, Stearamidopropyldimethyl(myristylacetat)-ammoniumchlorid oder einer Mischung davon bestehenden Gruppe; noch mehr bevorzugt handelt es sich bei dem Tensid von Formel (I) um Behenyltrimethylammoniumchlorid.

**3.** Zusammensetzung nach Anspruch 1 oder 2, wobei das Tensid von Formel (I) in einer Menge im Bereich von 0,01 bis 10 Gew.-%, vorzugsweise im Bereich von 0,05 bis 5 Gew.- % vorliegt, bezogen auf das Gesamtgewicht der Zusammensetzung.

**4.** Zusammensetzung nach einem der vorhergehenden Ansprüche 1 bis 3, wobei die trialkylsilylterminierten Dialkylpolysiloxane die Formel (II) haben:

$$R'_3SiO(R'_2SiO)pSiR'_3 \qquad \text{Formel (II)}$$

worin:

- R', gleich oder verschieden, ein einwertiger Kohlenwasserstoffrest mit 1 bis 18 Kohlenstoffatomen bzw. mit 1 bis 6 Kohlenstoffatomen ist, und
- p eine ganze Zahl von 500 bis 2000, vorzugsweise von 1000 bis 2000 ist;

und vorzugsweise Polydimethylsiloxane (PDMS) oder Dimethicone sind.

**5.** Zusammensetzung nach einem der vorhergehenden Ansprüche 1 bis 4, wobei die trialkylsilylterminierten Dialkylpolysiloxane eine Viskosität von 40.000 bis 70.000 mPa.s bei 25°C haben, mehr bevorzugt eine Viskosität von 51.000 bis 70.000 mPa.s bei 25°C.

**6.** Zusammensetzung nach einem der vorhergehenden Ansprüche 1 bis 5, wobei die Aminosilikone die Formel (III) haben:

$$XR_2Si(OSiAR)_n(OSiR_2)_mOSiR_2X \qquad \text{Formel (III)}$$

worin:

- R, gleich oder verschieden, ein einwertiger Kohlenwasserstoffrest mit 1 bis 18 Kohlenstoffatomen, vorzugsweise mit 1 bis 6 Kohlenstoffatomen ist,
- X, gleich oder verschieden, R oder eine Hydroxylgruppe (OH-Gruppe) oder eine $C_1$-$C_6$-Alkoxygruppe ist; vorzugsweise ist X R;
- A ein Aminorest der Formel $-R^1-[NR^2-R^3-]_xNR^2_2$ oder die protonierten Aminoformen des Aminorestes ist, wobei $R^1$ ein $C_1$-$C_6$-Alkylenrest ist, vorzugsweise ein Rest der Formel $-CH_2CH_2CH_2-$ oder $-CH_2CH(CH_3)CH_2-$, $R^2$, gleich oder verschieden, ein Wasserstoffatom oder ein $C_1$-$C_4$-Alkylrest ist, vorzugsweise ein Wasserstoffatom, $R^3$ ein $C_1$-$C_6$-Alkylenrest ist, vorzugsweise ein Rest der Formel $-CH_2CH_2-$, und x 0 oder 1 ist;
- m+n eine ganze Zahl von 50 bis etwa 1000 ist, vorzugsweise von 50 bis 600;

wobei A vorzugsweise ein Aminorest der Formel $-R^1-[NR^2-R^3-]_xNR^2_2$ ist, oder die protonierten Aminoformen des Aminorestes, wobei $R^1$ $-CH_2CH_2CH_2-$ oder $-CH_2CH(CH_3)CH_2-$ ist, $R^2$ Wasserstoffatome sind, $R^3$ $-CH_2CH_2-$ ist und x 1 ist.

**7.** Zusammensetzung nach einem der vorhergehenden Ansprüche 1 bis 6, wobei die Aminosilikone eine Viskosität von 1.500 bis 15.000 mPa.s haben; und/oder einen Aminwert von 3,5 bis 8 mg KOH pro Gramm Aminosilikon.

**8.** Zusammensetzung nach einem der vorhergehenden Ansprüche 1 bis 7, wobei die Silikonmischung (i) ein oder mehrere trialkylsilylterminierte Dialkylpolysiloxane mit einer Viskosität von 40.000 bis weniger als 100.000 mPa.s bei 25°C in einer Menge von 70 bis 90 Gew.-%, vorzugsweise von 75 bis 85 Gew.-% umfasst, und (ii) ein oder mehrere Aminosilikone mit einer Viskosität von 1.000 bis 15.000 mPa.s bei 25°C und einem Aminwert von 2 bis 10 mg KOH pro Gramm Aminosilikon, in einer Menge von 10 bis 30 Gew.-%, vorzugsweise von 15 bis 25 Gew.-% umfasst, bezogen auf das Gesamtgewicht der Silikonmischung.

**9.** Zusammensetzung nach einem der vorhergehenden Ansprüche 1 bis 8, wobei die Mischung von Emulgatoren einen oder mehrere Emulgatoren umfasst, die ausgewählt sind aus:

(i) Polyoxyalkylenalkylethern, insbesondere (poly)ethoxylierten Fettalkoholen der Formel: $R_3-(OCH_2CH_2)_cOH$, wobei

- $R_3$ eine lineare oder verzweigte $C_8$-$C_{40}$-Alkyl- oder -Alkenylgruppe, vorzugsweise eine $C_8$-$C_{30}$-Alkyl- oder -Alkenylgruppe repräsentiert, optional substituiert mit einer oder mehreren Hydroxylgruppen, und
- c eine ganze Zahl zwischen 1 und einschließlich 200 ist, vorzugsweise zwischen 2 und 150 und genauer gesagt zwischen 4 und 50, am meisten bevorzugt zwischen 8 und 20,

wobei die (poly)ethoxylierten Fettalkohole insbesondere Fettalkohole mit 8 bis 22 Kohlenstoffatomen sind, oxyethyleniert mit 1 bis 30 mol Ethylenoxid (1 bis 30 OE);

(ii) Polyoxyalkylen-($C_8$-$C_{32}$)-alkylphenylethern,
(iii) Polyoxyalkylensorbitan-($C_8$-$C_{32}$)-fettsäureestern, insbesondere polyethoxylierten Fettsäureestern von Sorbitan, die vorzugsweise von 2 bis 40 Ethylenoxideinheiten enthalten, am meisten bevorzugt von 2 bis 20 Ethylenoxideinheiten; vorzugsweise ausgewählt aus polyoxyethylenierten Sorbitan-($C_{10}$-$C_{24}$)-fettsäureestern, die vorzugswese von 2 bis 40 Ethylenoxideinheiten enthalten, am meisten bevorzugt von 2 bis 20 Ethylenoxideinheiten; und
(iv) polyoxyethylenierten ($C_8$-$C_{32}$)-Fettsäureestern, die zum Beispiel von 2 bis 150 mol Ethylenoxid enthalten; vorzugsweise aus polyoxyethylenierten ($C_{10}$-$C_{24}$)-Fettsäureestern, die zum Beispiel von 2 bis 150 mol Ethylenoxid enthalten.

**10.** Zusammensetzung nach einem der vorhergehenden Ansprüche 1 bis 9, wobei die Öl-in-Wasser-Emulsion Folgendes umfasst:

- die Mischung von Emulgatoren in einer Gesamtmenge von 5 bis 15 Gew.-%, vorzugsweise von 8 bis 15 Gew.-%, am meisten bevorzugt von 10 bis 12 Gew.-%, bezogen auf das Gesamtgewicht der Emulsion; und/oder
- nichtionische Emulgatoren in einer Gesamtmenge von 5 bis 15 Gew.-%, vorzugsweise von 8 bis 15 Gew.-%, am meisten bevorzugt von 10 bis 12 Gew.-%, bezogen auf das Gesamtgewicht der Emulsion; und/oder
- kationische Emulgatoren in einer Gesamtmenge von 0,5 bis 1,5 Gew.-%, bezogen auf das Gesamtgewicht der Emulsion; und/oder
- die Silikonmischung in einer Gesamtmenge von 40 bis 60 Gew.-%, vorzugsweise von 45 bis 55 Gew.-%, bezogen auf das Gesamtgewicht der Emulsion; und/oder
- das/die trialkylsilylterminierte(n) Dialkylpolysiloxan(e) in einer Gesamtmenge von 35 bis 45 Gew.-%, vorzugsweise von 38 bis 42 Gew.-%, bezogen auf das Gesamtgewicht der Emulsion; und/oder
- das/die Aminosilikon(e) in einer Gesamtmenge von 5 bis 15 Gew.-%, vorzugsweise von 8 bis 12 Gew.-%, bezogen auf das Gesamtgewicht der Emulsion; und/oder
- Wasser in einer Menge von 25 bis 50 Gew.-%, vorzugsweise von 30 bis 45 Gew.-%, am meisten bevorzugt von 35 bis 42 Gew.-%, bezogen auf das Gesamtgewicht der Emulsion.

**11.** Zusammensetzung nach einem der vorhergehenden Ansprüche 1 bis 10, wobei die Öl-in-Wasser-Emulsion (ii) eine Teilchengröße Dso von 100 bis 300 nm, vorzugsweise von 150 bis 250 nm, am meisten bevorzugt von 160 bis 200 nm hat, ausgedrückt in Volumen.

**12.** Zusammensetzung nach einem der vorhergehenden Ansprüche 1 bis 11, wobei die Öl-in-Wasser-Emulsion (ii) in einer Menge im Bereich von 0,1 bis 15 Gew.-%, vorzugsweise von 0,5 bis 10 Gew.-%, mehr bevorzugt von 1 bis 8 Gew.-%, noch mehr bevorzugt in einer Menge von 1,5 bis 5 Gew.-% vorliegt, bezogen auf das Gesamtgewicht der Zusammensetzung.

**13.** Zusammensetzung nach einem der vorhergehenden Ansprüche 1 bis 12, wobei der Fettalkohol aus der Verbindung der Formel (IV) ausgewählt ist:

R-OH Formel (IV)

worin:
R von 8 bis 30 Kohlenstoffatome, vorzugsweise eine $C_{12}$-$C_{24}$-Alkylgruppe oder eine $C_{12}$-$C_{24}$-Alkenylgruppe bezeichnet, wobei R optional mit einer oder mehreren Hydroxylgruppen, vorzugsweise mit einer oder zwei Hydroxylgruppen substituiert ist; mehr bevorzugt ist der Fettalkohol ausgewählt aus der aus Cetylalkohol, Stearylalkohol, Behenylalkohol, Isocetylalkohol, Isostearylalkohol, Isobehenylalkohol, Oleylalkohol und Mischungen davon bestehenden Gruppe; noch mehr bevorzugt ausgewählt aus der aus Cetylalkohol, Stearylalkohol oder einer Mischung davon bestehenden Gruppe.

**14.** Zusammensetzung nach einem der vorhergehenden Ansprüche 1 bis 13, wobei der Fettalkohol in einer Menge im

Bereich von 1 bis 15 Gew.-%, vorzugsweise von 2 bis 10 Gew.-% und mehr bevorzugt von 3 bis 8 Gew.-% vorliegt, bezogen auf das Gesamtgewicht der Zusammensetzung.

**15.** Zusammensetzung nach einem der vorhergehenden Ansprüche 1 bis 14, ferner mit mindestens einem zusätzlichen Silikon, ausgewählt aus Polyorganosiloxanen in Form von Ölen, Wachsen, Harzen oder Gummis; vorzugsweise ausgewählt aus Polydiorganosiloxanen mit einer hohen zahlengemittelten Molekülmasse zwischen 200.000 und 1.000.000, vorzugsweise zwischen 500.000 und 1.000.000, bei Verwendung allein oder als Mischung in einem Lösungsmittel.

**16.** Zusammensetzung nach einem der vorhergehenden Ansprüche 1 bis 15, wobei das zusätzliche Silikon in einer Menge im Bereich von 0,01 bis 10 Gew.-%, vorzugsweise von 0,05 bis 5 Gew.-% vorliegt, bezogen auf das Gesamtgewicht der Zusammensetzung.

**17.** Verfahren für die kosmetische Behandlung von Haar, vorzugsweise zum Konditionieren von Haar, mit den Schritten des Aufbringens der Zusammensetzung nach einem der Ansprüche 1 bis 16 auf das Haar, vorzugsweise im nassen Zustand, und optional des Ausspülens mit Wasser nach einer optionalen leave-on-Zeit.

**Revendications**

**1.** Composition capillaire comprenant :

(i) au moins un tensioactif cationique de formule (I),

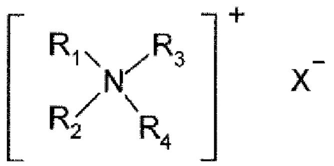

(I)

dans laquelle :

$R_1$ à $R_4$, identiques ou différents, représentent un groupe aliphatique linéaire ou ramifié comprenant de 1 à 30 atomes de carbone, un groupe aromatique tel qu'aryle ou alkylaryle,
au moins l'un des groupes $R_1$ à $R_4$ comprenant de 8 à 30 atomes de carbone, et
$X^-$ est un contre-ion anionique choisi parmi des halogénures ;

(ii) une émulsion huile-dans-l'eau ayant une taille de particules $D_{50}$ inférieure à 350 nm, exprimée en volume et mesurée avec la méthode de spectroscopie par corrélation de photons (PCS), et comprenant :

- un mélange de silicones comprenant (i) un dialkylpolysiloxane à terminaison trialkylsilyle ayant une viscosité de 40 000 à moins de 100 000 mPa.s à 25 °C et un taux de cisaillement de 1 $s^{-1}$, et (ii) une amino silicone ayant une viscosité de 1000 à 15 000 mPa.s à 25 °C à un taux de cisaillement de 1 $s^{-1}$, et un indice d'amine de 2 à 10 mg de KOH par gramme d'amino-silicone, les viscosités étant déterminées avec les méthodes à rhéomètre rotatif de la convention de la pharmacopée américaine (USP) 912 ;
- un mélange d'émulsifiants comprenant un ou plusieurs émulsifiants non ioniques, dans laquelle le mélange d'émulsifiants a une HLB de 10 à 16 ; et
- de l'eau ; et

(iii) au moins un alcool gras en $C_{8-40}$.

**2.** Composition selon la revendication 1, dans laquelle dans la formule (I), au moins l'un des groupes $R_1$ à $R_4$ comprend de 12 à 24 atomes de carbone, dans laquelle les groupes aliphatiques peuvent comprendre des hétéroatomes, de préférence de l'oxygène, de l'azote, du soufre ou des halogènes ; de préférence le tensioactif de la formule (I) est sélectionné dans le groupe constitué d'halogénures de tétraalkylammonium dans lesquels le groupe alkyle contient de 12 à 22 atomes de carbone, ou un mélange de ceux-ci ; de manière davantage préférée sélectionné dans le

groupe constitué d'un chlorure de béhényltriméthylammonium, d'un chlorure de distéaryldiméthylammonium, d'un chlorure de cétyltriméthylammonium, d'un chlorure de benzyldiméthylstéarylammonium, d'un chlorure d'hydroxyéthyl oléyldiméthylammonium, d'un chlorure de palmitylamidopropyltriméthylammonium, d'un chlorure de stéaramidopropyldiméthyl(acétate de myristyle)ammonium, ou d'un mélange de ceux-ci ; de manière encore davantage préférée le tensioactif de formule (I) est un chlorure de béhényltriméthylammonium.

3. Composition selon la revendication 1 ou 2, dans laquelle le tensioactif de formule (I) est présent en une quantité dans la page de 0,01 à 10 % en poids, de préférence de 0,05 % à 5 % en poids, par rapport au poids total de la composition.

4. Composition selon l'une quelconque des revendications précédentes 1 à 3, dans laquelle les dialkylpolysiloxanes à terminaison trialkylsilyle sont de formule (II) :

$$R'_3SiO(R'_2SiO)_pSiR'_3 \quad \text{formule (II)}$$

dans laquelle :

- R', identique ou différent, est un radical hydrocarbure monovalent ayant de 1 à 18 atomes de carbone, de 1 à 6 atomes de carbone, et
- p est un nombre entier de 500 à 2000, de préférence de 1000 à 2000 ;

et de préférence sont des PDMS (polydiméthylsiloxanes ou diméthicones) .

5. Composition selon l'une quelconque des revendications précédentes 1 à 4, dans laquelle les dialkylpolysiloxanes à terminaison trialkylsilyle ont une viscosité de 40 000 à 70 000 mPa.s à 25 °C de manière davantage préférée une viscosité de 51 000 à 70 000 mPa.s à 25 °C.

6. Composition selon l'une quelconque des revendications précédentes 1 à 5, dans laquelle les amino silicones sont de formule (III) :

$$XR_2Si(OSiAR)_n(OSiR_2)_mOSiR_2X \quad \text{formule (III)}$$

dans laquelle :

- R, identique ou différent, est un radical hydrocarbure monovalent ayant de 1 à 18 atomes de carbone, de préférence de 1 à 6 atomes de carbone,
- X, identique ou différent, est R ou un groupe hydroxyle (OH) ou alcoxy en $C_1$-$C_6$ ; de préférence X est R ;
- A est un radical amino de formule $-R^1-[NR^2-R^3-]_xNR^2_2$, ou les formes amino protonées dudit radical amino, dans lequel $R^1$ est un radical alkylène en $C_1$-$C_6$, de préférence un radical de formule $-CH_2CH_2CH_2-$ ou $-CH_2CH(CH_3)CH_2-$, $R^2$, identique ou différent, est un atome d'hydrogène ou un radical alkyle en $C_1$-$C_4$, de préférence un atome d'hydrogène, $R^3$ est un radical alkylène en $C_1$-$C_6$, de préférence un radical de formule $-CH_2CH_2-$, et x est 0 ou 1 ;
- m + n est un nombre entier de 50 à environ 1000, de préférence de 50 à 600 ;
de préférence, A étant un radical amino de formule $-R^1-[NR^2-R^3-]_xNR^2_2$, ou les formes amino protonées dudit radical amino, dans lequel $R^1$ est $-CH_2CH_2CH_2-$ ou $-CH_2CH(CH_3)CH_2-$, les $R^2$ sont des atomes d'hydrogène, $R^3$ est
- $CH_2CH_2$-, et x est 1.

7. Composition selon l'une quelconque des revendications précédentes 1 à 6, dans laquelle les amino silicones ont une viscosité de 1500 à 15 000 mPa.s à 25 °C ; et/ou un indice d'amine de 3,5 à 8 mg de KOH par gramme d'amino silicone.

8. Composition selon l'une quelconque des revendications précédentes 1 à 7, dans laquelle le mélange de silicones comprend (i) un ou plusieurs dialkylpolysiloxanes à terminaison trialkylsilyle ayant une viscosité de 40 000 à moins de 100 000 mPa.s à 25 °C en une quantité de 70 à 90 % en poids, de préférence de 75 à 85 % en poids et (ii) une ou plusieurs amino silicones ayant une viscosité de 1000 à 15 000 mPa.s à 25 °C et un indice d'amine de 2 à 10 mg de KOH par gramme d'amino silicone, en une quantité de 10 à 30 % en poids, de préférence de 15 à 25 % en poids, par rapport au poids total du mélange de silicones.

**9.** Composition selon l'une quelconque des revendications précédentes 1 à 8, dans laquelle le mélange d'émulsifiants comprend un ou plusieurs émulsifiants choisis parmi :

(i) des éthers d'alkyle polyoxyalkylénés, notamment des alcools gras (poly)éthoxylés de formule : $R_3$-$(OCH_2CH_2)_cOH$ avec :

- $R_3$ représentant un groupe alkyle en $C_8$-$C_{40}$ ou alcényle, de préférence un groupe alkyle en $C_8$-$C_{30}$ ou alcényle, facultativement substitué par un ou plusieurs groupes hydroxyles, et
- c étant un nombre entier entre 1 et 200 inclus, préférentiellement entre 2 et 150 et plus particulièrement entre 4 et 50, de manière préférée entre toutes entre 8 et 20,

les alcools gras (poly)éthoxylés sont plus particulièrement des alcools gras comprenant de 8 à 22 atomes de carbone, oxyéthylénés avec 1 à 30 mol d'oxyde d'éthylène (1 à 30 OE) ;

(ii) des éthers d' (alkyl en $C_8$-$C_{32}$) phényle polyoxyalkylénés,
(iii) des esters d'acide gras en $C_8$-$C_{32}$ de sorbitane polyoxyalkylénés, notamment des esters d'acide gras de sorbitane polyéthoxylés contenant de préférence de 2 à 40 unités oxyde d'éthylène, de manière préférée entre toutes de 2 à 20 unités oxyde d'éthylène ; de préférence des esters d'acide gras en $C_{10}$-$C_{24}$ de sorbitane polyoxyéthylénés de préférence contenant de 2 à 40 unités oxyde d'éthylène, de manière préférée entre toutes de 2 à 20 unités oxyde d'éthylène ; et
(iv) des esters d'acide gras en $C_8$-$C_{32}$ polyoxyéthylénés contenant par exemple de 2 à 150 mol d'oxyde d'éthylène ; de préférence des esters d'acide gras en $C_{10}$-$C_{24}$ polyoxyéthylénés contenant par exemple de 2 à 150 mol d'oxyde d'éthylène.

**10.** Composition selon l'une quelconque des revendications précédentes 1 à 9, dans laquelle l'émulsion huile-dans-l'eau comprend :

- le mélange d'émulsifiants en une quantité totale de 5 à 15 % en poids, de préférence de 8 à 15 % en poids, de manière préférée entre toutes de 10 à 12 % en poids, par rapport au poids total de l'émulsion ; et/ou
- des émulsifiants non ioniques en une quantité totale de 5 à 15 % en poids, de préférence de 8 à 15 % en poids, de manière préférée entre toutes de 10 à 12 % en poids, par rapport au poids total de l'émulsion ; et/ou
- des émulsifiants cationiques en une quantité totale de 0,5 à 1,5 % en poids, par rapport au poids total de l'émulsion ; et/ou
- le mélange de silicones en une quantité totale de 40 à 60 % en poids, de préférence de 45 à 55 % en poids, par rapport au poids total de l'émulsion ; et/ou
- le(s) dialkylpolysiloxane(s) à terminaison trialkylsilyle en une quantité totale de 35 à 45 % en poids, de préférence de 38 à 42 % en poids, par rapport au poids total de l'émulsion ; et/ou
- l'amino silicone (les amino silicones) en une quantité totale de 5 à 15 % en poids, de préférence de 8 à 12 % en poids, par rapport au poids total de l'émulsion ; et/ou
- de l'eau en une quantité totale de 25 à 50 % en poids, de préférence de 30 à 45 % en poids, de manière préférée entre toutes de 35 à 42 % en poids, par rapport au poids total de l'émulsion.

**11.** Composition selon l'une quelconque des revendications précédentes 1 à 10, dans laquelle l'émulsion huile-dans-l'eau (ii) a une taille de particules $D_{50}$ inférieure de 100 à 300 nm, de manière davantage préférée de 150 à 250 nm, de manière préférée entre toutes de 160 à 200 nm, exprimée en volume.

**12.** Composition selon l'une quelconque des revendications précédentes 1 à 11, dans laquelle l'émulsion huile-dans-l'eau (ii) est présente en une quantité dans la page de 0,1 à 15 % en poids, de préférence de 0,5 % à 10 % en poids, de manière davantage préférée de 1 % à 8 % en poids, de manière encore davantage préférée de 1,5 % à 5 % en poids, par rapport au poids total de la composition.

**13.** Composition selon l'une quelconque des revendications précédentes 1 à 12, dans laquelle l'alcool gras est sélectionné parmi le composé de formule (IV) :

R-OH formule (IV)

dans laquelle :
R indique de 8 à 30 atomes de carbone, de préférence un groupe alkyle en $C_{12}$-$C_{24}$ ou un groupe alcényle en $C_{12}$-$C_{24}$, dans laquelle R est facultativement substitué par un ou plusieurs groupes hydroxyle, de préférence par

un ou deux groupes hydroxyle ; de manière davantage préférée, l'alcool gras est sélectionné dans le groupe constitué d'un alcool cétylique, d'un alcool stéarylique, d'un alcool béhénylique, d'un alcool isocétylique, d'un alcool isostéarylique, d'un alcool isobéhénylique, d'un alcool oléylique, et de mélanges de ceux-ci ; de manière encore davantage préférée sélectionné dans le groupe constitué d'un alcool cétylique, d'un alcool stéarylique, ou d'un mélange de ceux-ci.

**14.** Composition selon l'une quelconque des revendications précédentes 1 à 13, dans laquelle l'alcool gras est présent en une quantité dans la page de 1 % à 15 %, de préférence de 2 % à 10 % et de manière davantage préférée de 3 % à 8 % en poids par rapport au poids total de la composition.

**15.** Composition selon l'une quelconque des revendications précédentes 1 à 14, comprenant en outre au moins une silicone supplémentaire, choisie parmi des polyorganosiloxanes sous la forme d'huiles, de cires, de résines ou de gommes ; de préférence sélectionnée parmi des polydiorganosiloxanes ayant des masses moléculaires moyennes en nombre élevées comprises entre 200 000 et 1 000 000, de préférence entre 500 000 et 1 000 000, utilisée seule ou sous la forme d'un mélange dans un solvant.

**16.** Composition selon l'une quelconque des revendications précédentes 1 à 15, dans laquelle la silicone supplémentaire est présente en une quantité dans la plage de 0,01 % à 10 % en poids, de préférence de 0,05 % à 5 % en poids, par rapport au poids total de la composition.

**17.** Procédé pour le traitement cosmétique des cheveux, de préférence pour le conditionnement des cheveux, comprenant les étapes d'application aux cheveux, de préférence dans un état humide, de la composition selon une quelconque des revendications 1 à 16, et facultativement leur rinçage à l'eau après un temps de pose facultatif.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6417145 B
- US 6610280 B


### Non-patent literature cited in the description


- **GRIFFIN, J.** *Soc. Cosm. Chem.,* 1954, vol. 5 (4), 249-256
- Galenica 5: Les systèmes disperses - Tome I - Agents de surface et émulsions. **F. PUISIEUX ; M. SEILLER.** Notions de HLB et de HLB critique. 153-194
- **DAVIES JT.** A quantitative kinetic theory of emulsion type, I. Physical chemistry of the emulsifying agent. *Gas/Liquid and Liquid/Liquid Interface (Proceedings of the International Congress of Surface Activity),* 1957, 426-438
- **GODFREY, J.** Cationic emulsifiers in cosmetics. *Soc. Cosmetic Chemists,* 1966, vol. 17, 17-27
- **WALTER NOLL'S.** Chemistry and Technology of Silicones. Academic Press, 1968